# EUROPEAN PATENT APPLICATION

(11) **EP 2 452 652 A1**
(43) Date of publication of application: **16.05.2012**
(21) Application number: 10190621.2
(22) Date of filing: 10.11.2010
(51) Int. Cl.: A61C 19/04, A61B 5/00, A61B 6/03, A61B 6/14

(54) **Non-invasive method for semi-automatic detection and measurement of periodontal bone defects**

(71) Applicant: Straumann Holding AG, 4002 Basel (CH)
(72) Inventor: Schnappauf, Albrecht, 4052 Basel (CH); Fleiner, Jonathan, 78464 Konstanz (DE); Schulze, Dirk, 79098 Freiburg (DE)
(74) Representative: Modiano, Micaela Nadia

(57) **Abstract**

The present invention relates to a method and a device for non-invasive semi-automatic detection and measurement of periodontal bone defects based on computer tomography (CT) or digital volume tomography (DVT) scan data, the method comprising the following steps:
a) receiving the CT or DVT scan data of a jawbone with teeth (10);
b) displaying a part of the jawbone with the teeth (10) using the CT or DVT scan data;
c) adding to the CT or DVT scan data within at least one cross-section of the tooth (10) and the jawbone along a middle reference axis (A0) of the tooth (10) predefined landmarks at measuring positions on a neck of tooth (11) and at the periodontal bone (15), respectively;
d) calculating the periodontal bone defect at each measuring position by said respective landmarks, and thus providing for each measuring position a value of the respective periodontal bone defect as an index of the respective periodontal bone condition;
e) indicating the periodontal bone defects within the display.

## Description

### Field of the Invention

The present invention relates to a non-invasive method and a device for semiautomatic detection and measurement of periodontal bone defects. In particular said non-invasive method is based on 3D-CT or -DVT scan data of a jawbone with respective teeth.

### Background of the Invention / State of the art

Periodontal disease called periodontitis or periodontosis leads to increasing periodontal bone defects which go along with an alveolar bone destruction resulting in smaller and less stable tooth sockets. This disease progresses slowly over a long period of time without showing any symptoms and it is often not treated until a patient unexpectedly has suffered serious unrecoverable damage. Further, periodontal disease is a more frequent cause of tooth loss than tooth decay in people in their forties or older.

The periodontal bone defects can be discerned and measured by depth measurement of a respective groove between the tooth and the surrounding gingival tissue, said groove being called sulcus gingivae. The depth measurement of the sulcus gingivae is done by a dental instrument like a little ruler or a tip with markers along the tip which gets inserted into the sulcus gingivae with little pressure and read off. For reference, a clinically normal sulcus gingivae can comprise a groove with a depth of 0.5-2.0 mm.

US2009/0062383 discloses a dental clinical instrument for diagnosis of periodontal disease. The dental clinical instrument comprises a main body held by hand for application of a certain force during diagnosis, a probe having a front portion in the form of a tip bent downward to be inserted into the periodontal groove as the sulcus gingivae to be diagnosed and a rear portion coupled to the main body to allow the front portion to be movable up and down. A pressure indicator displays the force applied to the front portion, so that during the insertion of the tip into the sulcus gingivae the insertion force for the depth measurement can be controlled. The depth gets measured visually by markers along the tip. Due to this configuration, a user can easily discern the force applied to the probe during the diagnosis of the periodontal bone defect and a diagnostic error arising from a different insertion force of another user can be significantly reduced.

JP2007152004 discloses an apparatus for periodontal measurement, whose small-sized and high-precision probe safely and automatically achieves conventional sulcus gingivae measurement under invasive and traditional fixed pressures, using a probe tip of a gel-like elastomer whose external tip surface is coated with a reflective means. The tip is supplied with light through fibers, and its reflective light is detected by a photo-diode for measurement of the displacement. The necessary pressures of the tip insertion into the sulcus gingivae are described to be smaller than conventional pressures of conventional other dental instruments designed for this purpose.

CN1184625 and the related German Patent DE19755169 disclose a dental measuring device for periodontal bone defects which comprises a main hand-held part with an actuating element slidable along the hand-held part and a probe tip which has the function of a sleeve. The actuating element slidable along the hand-held part is connected with a flexible tip sliding longitudinally within and along the probe sleeve and protruding out of the probe sleeve as far as the actuating element is slid along the hand-held part. The protrusion of the flexible tip is to be adapted to the depth of the sulcus gingivae, while the user discerns its depth reading off the sliding distance of the actuating element on the main hand-held part.

All these methods are invasive and therefore not really convenient and comfortable for the patient so that preventive diagnosis is often done too little and too late. Also the fact that different pressures during the insertion of the measuring tip into the sulcus gingivae lead to different results is an obvious disadvantage.

### Summary of the invention

The purpose of the invention is to overcome the shortcomings explained above and to provide a non-invasive method for semi-automatic detection and measurement of periodontal bone defects using 3D-CT or -DVT scan data of a jawbone with teeth.

Another purpose of the invention is to provide a device for an implementation of the above mentioned method.

The above purposes as well as further purposes/objectives which will become apparent from the following description are achieved by the features mentioned in the independent claims. Additional advantageous features and characteristics of the invention are mentioned in the dependent claims.

The invention is set forth and characterized in the main claims, while dependent claims describe other advantageous characteristics of the invention.

One solution of a preferred embodiment according the present invention is disclosed in the following drawings with detailed description but it shall not be limiting the invention.

### Brief description of the drawings

- Fig. 1 an bone: is a cross-section side view of a tooth within its socket of alveolar bone and periodontal ligament, showing periodontal defects on the right and on the left side.
- Fig. 2: is the same cross-section side view as within Fig. 1 but with added predefined landmarks.
- Fig. 3 to bone: is a drawing of a part of the cross-section side view similar Fig. 2 with the landmarks on the right side and periodontal defect measurements.
- Fig. 4 of left: is a picture of a display showing the teeth with indications the periodontal bone defects at some teeth and a table on the side with values of the periodontal bone defects.
- Fig. 5 of: is a picture of the display with a panoramic view of the teeth an upper jaw with measurement results of the periodontal bone defects and with the possibility of further selections of commands.

### Detailed description of a preferred method and an embodiment

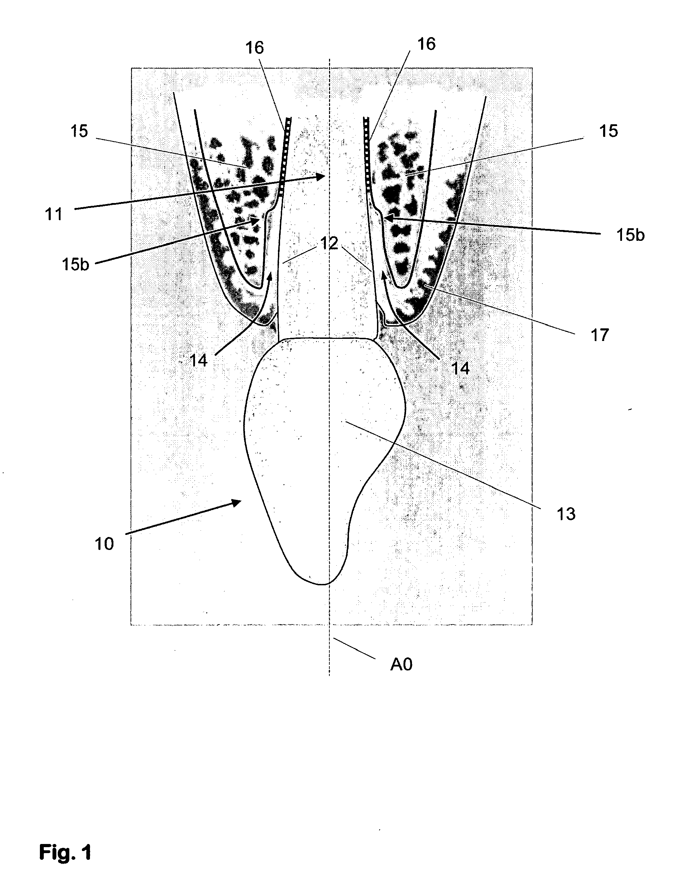

Fig. 1 shows based on 3D computer tomography (CT) or digital volume tomography (DVT) scan data of a jaw bone of a patient a cross-section side view of a tooth 10 comprising a neck of tooth 11 with its edge 12 and an enamel 13, wherein the neck of tooth 11 is engaged and held within the alveolar bone 15 having the form of a socket with a thin layer of periodontal ligament 16 between the neck of tooth 11 and the alveolar bone 15. The alveolar bone 15 is covered and shielded by a gingiva 17 ending with its distal part at the neck of tooth 11, in this case. The alveolar bone 15 shows in the area of its distal part next to the tooth 10 an alveolar bone destruction 15b leading to a sulcus gingivae 14 which is in the shape of a pocket around the tooth 10 between the neck of tooth 11 and the alveolar bone 15 and the gingiva 17, respectively. The alveolar bone destruction 15b and the sulcus gingivae 14 is an alveolar bone defect causing a less stable footing of the tooth 10 within the alveolar bone 15. The depth of the sulcus gingivae 14 is taken as a measuring index for the periodontal defect and alveolar bone condition, respectively.

For detection and measurement of the alveolar bone defect and therewith of the alveolar bone condition, respectively, at the teeth 10 of a patient 3D-CT or -DVT scan data of the jawbones with teeth 10 are received and analyzed. Preferably a dental panoramic arch curve of an upper and/or of a lower jaw with teeth 10 is displayed (not shown here) wherein cross-sections of the teeth 10 and preferably along a middle reference axis A0 can be calculated and displayed, the cross-section of the tooth 10 showing also the neck of tooth 11 and its fit within the socket of the alveolar bone 15, as depicted in Fig. 1. Different cross-sections through the middle of and along the middle reference axis A0 of the tooth 10 are made showing different positions of the alveolar bone 15 for detection and measurement of possible alveolar bone defects.

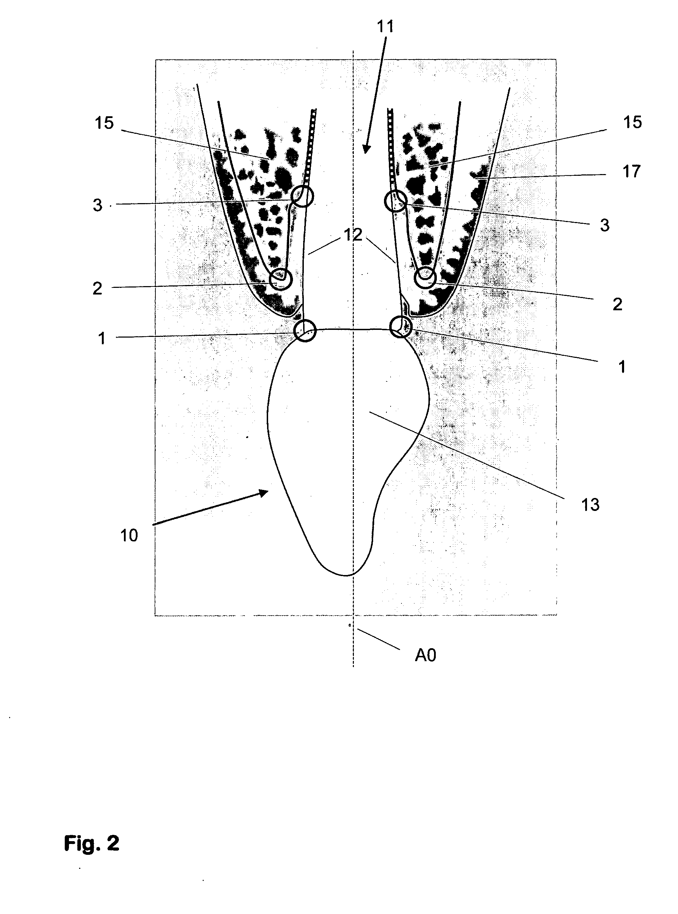

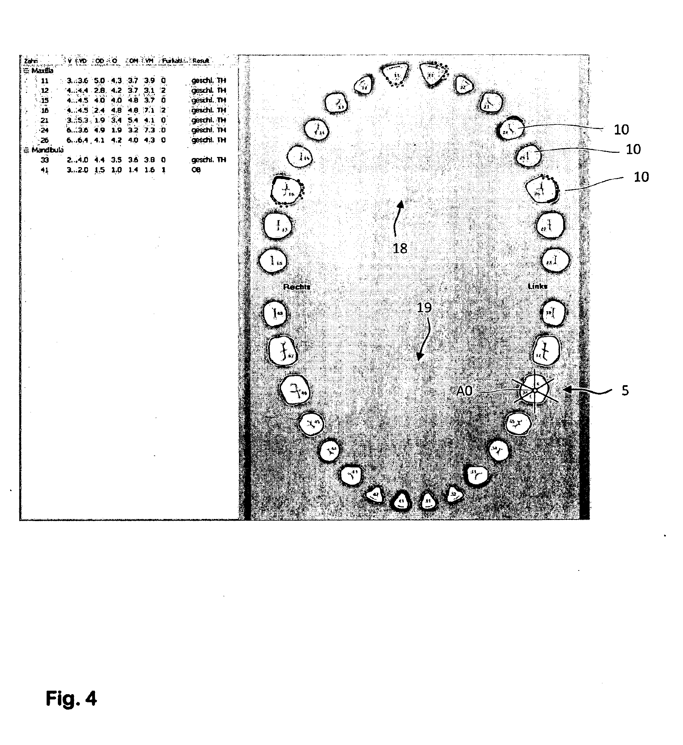

For each tooth 10 the middle reference axis A0 in the middle of and along said tooth 10 is defined. Cross-section views as for instance within Fig. 1 and Fig. 2 include said middle reference axis A0. Preferably the middle reference axis A0 is taken for automatic or semi-automatic generation of cross-section cuts through the tooth 10 and along said middle reference axis A0 resulting in cross-section cuts under different angles within a plane perpendicular to said middle reference axis A0 which is a top view of the tooth 10. In Fig. 4 at a position indicated with reference numeral 5 the tooth 10 in top view and 3 cross-section cuts under an increasing angle of 120° are shown.

In this way different cross-sections of the tooth 10 and including the middle reference axis A0 can be automatically or semiautomatically generated. Such cross-sections and middle reference axes give at respective different positions around the neck of tooth 11 and the distal part of the alveolar bone 15 a view and a measurement possibility of the respective periodontal bone defect. Each of said cross-sections of the tooth 10 shows on opposite sides with respect to the middle reference axis A0 the condition of the periodontal bone defect which will be measured in the following. Preferably three cross-sections are used, preferably under an angle of 0°, of 120° and of 240°, which provide together 6 measuring positions. A higher spatial resolution with respect to measuring positions is also imaginable and is preferably selectable.

Fig. 2 shows the same cross-section view as Fig. 1, but now there are landmarks for the measuring and calculation of the periodontal bone defect added. As mentioned before, for each cross-section view there are preferably two measuring positions available, one on the right and one on the left side of the middle reference axis A0 at the respective alveolar bone 15 and the tooth 10. The landmarks are predefined as a first landmark 1 placed at the respective edge 12 of the neck of tooth 11 at a distal point merging with the enamel 13, a second landmark 2 placed at the distal outermost tip of the respective alveolar bone 15 on the right and on the left side of the tooth 10, and a third landmark 3 placed at the proximal innermost part of the respective sulcus gingivae where the alveolar bone 15 begins to align itself again with the edge 12 of the neck of tooth 11. A positioning of said first landmark 1, second landmark 2 and third landmark 3 and adding to the scan data is preferably done manually with expert skills within a displayed image, but an automatic positioning is also imaginable and possible by respective pattern recognition algorithms which is state of the art in other cases.

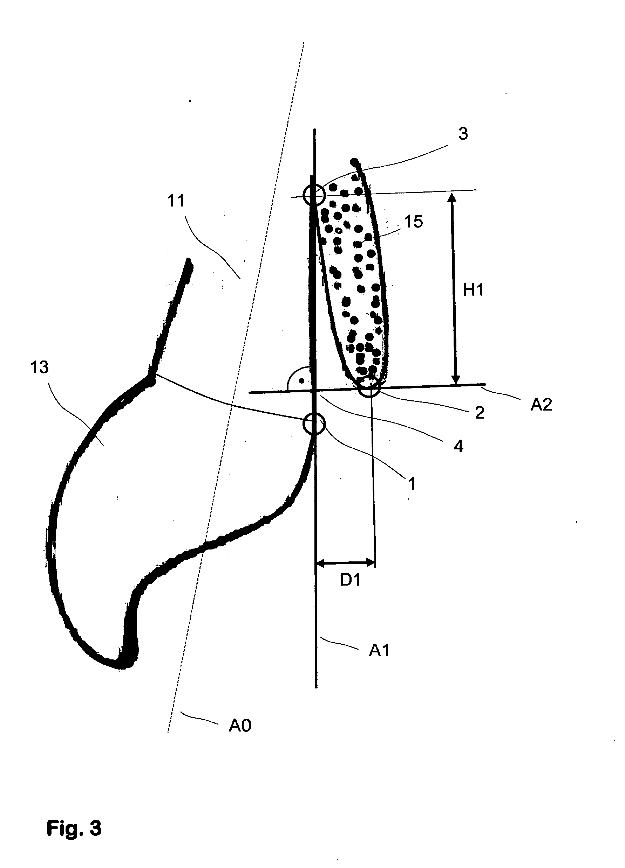

Fig. 3 shows a part of the cross-section view of Fig. 2 with the added landmarks 1-3 on the right side of the tooth 10, wherein the tooth 10 with its middle reference axis A0 is slightly tilted to the right side. The measurement of the periodontal defect is now done by measurement respectively by calculation of the depth H1 of the sulcus gingivae 14. Therefore a first line A1 is defined between the first landmark 1 and the third landmark 3, and a second line A2 is defined including the landmark 2 and being perpendicular to the first line A1 intersecting the first line A1 at an intersection point 4. The depth H1 of the sulcus gingivae 14 can now be calculated as the section of the line A1 between the third landmark 3 and the intersection point 4. The value of the depth H1 is taken as the value for the alveolar bone defect or the alveolar bone condition, respectively.

Another parameter, such as a shortest distance D1 between the neck of tooth 11 and the tip of the alveolar bone, is preferably also of interest and is preferably taken into account for the calculation of the alveolar bone defect and the alveolar bone condition, respectively.

The resulting values of the calculated alveolar bone defects are preferably indicated within the display of images of different views of the 3D-CT/-DVT scan data.

Fig. 4 is an image of the display showing the teeth 10 of the upper jaw 18 and of the lower jaw 19 each in top view perspective (of the respective teeth) and with respective indications of the values of the periodontal bone defects around each tooth 10 (only some teeth are indicated with reference numerals). The periodontal bone defects at the measured positions around each tooth 10 are preferably displayed in color around the respective tooth 10, wherein for instance a yellow color (dotted line) indicates a medium alveolar bone defect and a red color (full bold line) indicates a high alveolar bone defect. Other kinds of indications, such as other colors, shapes and highlighting are also imaginable and shall not be excluded.

On the left side of the display a table giving an overview of the measurement values of the periodontal bone defects is preferably displayed, showing preferably an average and a maximum value of the periodontal bone defects for the respective tooth 10.

For clarity reasons the position with reference numeral 5 shows the tooth 10 with three preferred cross-sections of the tooth 10 and along the middle reference axis A0 as planes perpendicular to the image. Said cross-sections provide together 6 measurement positions at the intersections between the respective planes and the edge of the round tooth 10, each measurement position comprising a first 1, a second 2 and a third landmark 3 (not shown).

The current invention shall not be limited to any kind of displaying the 3D-CT/-DVT scan data for the selection of the tooth 10 and/or the selection of the respective cross-section and/or the adding of the respective landmarks 1-3. Various kinds of displaying and selection possibilities are imaginable, for instance by clicking with a mouse on a certain tooth 10 within the display, such that the tooth 10 is chosen for the further selection of the respective cross-sections. Furthermore automatically added and displayed landmarks 1-3 can be manually corrected, the cross-section views of the respective tooth 10 with cross-sections are displayed and can be changed, for instance.

It is preferred that after reception of the 3D-CT/DVT scan data all the above mentioned steps are executed automatically by provision of respective pattern analysis algorithms, until the measuring results are displayed, whereupon for instance the respective middle reference axis A0 and the respective landmarks 1-3 can be manually corrected.

Moreover, the present invention provides additional software tools for the selection and the display of the 3D-CT/-DVT scan data or parts thereof and of all possible cross-sections and for the display of measuring results. These software tools are state of the art, for instance including zooming, selections via keyboard, mouse or touch screen, command inputs, using the mouse, overlapping and/or overlaying of display windows, coloring, indicating and printing as well as import and export functions for all kinds of actual formats, and the like.

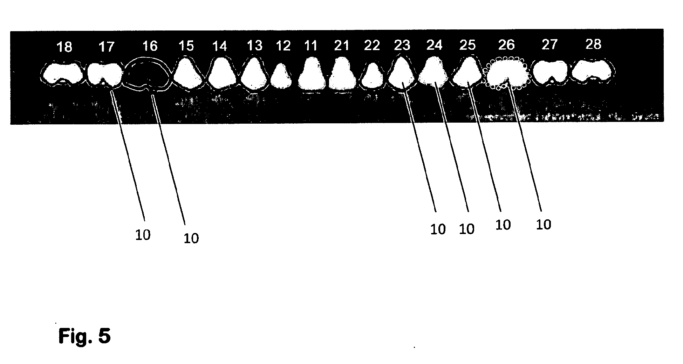

Fig. 5 shows another possible display of the measurement results of the upper jaw 18 in panorama view with indication of the measurement results of the periodontal bone defects around the teeth 10. A similar second parallel panorama view of the lower jaw 19 is also imaginable. In this display state the user can preferably select a tooth 10 by a mouse click in order to select further options for said tooth 10, for instance to select a certain cross-section view around the middle reference axis A0, or to select a rotation angle of the tooth 10 itself and the like.

Moreover, the present invention foresees the provision of a PC-based device, wherein 3D-CT/DVT scan data of the patient's jaw preferably in DICOM format are receivable and wherein the above non-invasive method for the semi-automatic detection and the measurement of the periodontal bone defects is implemented and usable.

Furthermore, although the present invention has been described in connection with certain specific methods, an embodiment and algorithms for instructional purposes, the present invention is not limited thereto. Accordingly various modifications, adaptations and combinations of various features of the described methods and the embodiment can be practiced without departing from the scope of the present invention as set forth in the accompanying claims.

Where technical features mentioned in any claim are followed by reference signs, those reference signs have been included for the sole purpose of increasing intelligibility of the claims and accordingly, such reference signs do not have any limiting effect on the scope of each element identified by way of example by such reference signs.

### List of Reference Numerals

- 1: first landmark
- 2: second landmark
- 3: third landmark
- 4: intersection point
- 5: position
- 10: tooth
- 11: neck of tooth
- 12: edge of the neck of tooth
- 13: enamel
- 14: sulcus gingivae
- 15: alveolar bone
- 15b: alveolar bone destruction
- 16: periodontal ligament
- 17: gingiva
- 18: upper jaw
- 19: lower jaw
- A0: middle reference axis of the tooth
- A1: first line
- A2: second line
- H1: section of the first line
- D1: shortest distance

## Claims

1. Method for non-invasive semi-automatic detection and measurement of periodontal bone defects based on computer tomography (CT) or digital volume tomography (DVT) scan data, the method comprising the following steps:
a) receiving the CT or DVT scan data of a jawbone with teeth (10);
b) displaying a part of the jawbone with the teeth (10) using the CT or DVT scan data;
c) adding to the CT or DVT scan data within at least one cross-section of the tooth (10) and the jawbone along a middle reference axis (A0) of the tooth (10) predefined landmarks at measuring positions on a neck of tooth (11) and at the periodontal bone (15), respectively;
d) calculating the periodontal bone defect at each measuring position by said respective landmarks, and thus providing for each measuring position a value of the respective periodontal bone defect as an index of the respective periodontal bone condition;
e) indicating the periodontal bone defects within the display.

2. Method according to claim 1, wherein in step b) the jawbone and the teeth (10) are displayed in 3D format and/or as 2D cross-section views.

3. Method according to claim 1 or 2, wherein in step c) as predefined landmarks at each measuring position the following are added:
- a first landmark (1) at an intersection between a proximal end of the enamel (13) and an edge (12) of the neck of tooth (11);
- a second landmark (2) at a distal outermost tip of the periodontal bone (15) next to the first landmark (1); and
- a third landmark (3) at a proximal innermost region of the periodontal bone (15) and of the sulcus gingivae (14) at a point where the neck of tooth (11) begins to engage/disengage with the alveolar bone (15),
wherein the first (1), second (2) and third landmark (3) span a plane which includes the middle reference axis (A0), the middle reference axis (A0) being the innermost longitudinal axis of the tooth (10).

4. Method according to claim 1 or 2, wherein as predefined landmarks within the cross-section of the tooth (10) the following are added:
- a first pair of landmarks (1), each first landmark (1) at an intersection between a proximal end of the enamel (13) and an edge (12) of the neck of tooth (11);
- a second pair of landmarks (2), each second landmark (2) at a distal outermost tip of the periodontal bone (15) next to the first landmark (1); and
- a third pair of landmarks (3), each third landmark (3) at a proximal innermost region of the periodontal bone (15) and of the sulcus gingivae (14) at a point where the neck of tooth (11) begins to engage/disengage with the alveolar bone (15),
wherein the first (1), second (2) and third pair of landmarks (3) span a plane which includes the middle reference axis (A0), the middle reference axis (A0) being the innermost longitudinal axis of the tooth (10), and wherein in step c) the predefined landmarks are placed within the cross-section of the tooth (10) on a right and on a left side of the neck of tooth (11) and at the periodontal bone (15), respectively.

5. Method according to one or more of the preceding claims, wherein in step c) the selection of the cross-section of the tooth (10) and the adding of the respective landmarks (1-3) are done manually, and/or wherein multiple cross-sections of multiple teeth (10) are selectable.

6. Method according to one or more of the preceding claims, wherein in step c) the selection of the cross-section of the tooth (10) and the adding of the respective first (1), second (2) and third landmarks (3) are done automatically by respective pattern recognition algorithms, said recognition algorithms further include the steps of:
- detecting the teeth (10);
- detecting the respective middle reference axes (A0)of the teeth (10) ;
- generating the respective cross-sections at predefined angles and
- detecting the respective positions of the landmarks (1-3), wherein the automatic adding is preferably done interactively with selection and command inputs.

7. Method according to one or more of the preceding claims, wherein in step c) the selection of the cross-sections of the tooth (10) is done in preselected angles, such as preferably 0°, 120° and 240°, or in freely selectable angles.

8. Method according to claim 5, wherein step c) further comprises the possibility of a manual correction of the automatically added first (1), second (2) and third landmarks (3).

9. Method according to one or more of claims 3 to 8, wherein in step d) the calculation of the periodontal bone defect at the respective measuring position is done by measuring the depth of the sulcus gingivae (14) by calculation of a section (H1) of a first line (A1) beginning at the third landmark (3) leading to the first landmark (1) and ending at an intersection (4) between the first line (A1) and a second line (A2) which is perpendicular to the first line (A1) and includes the second landmark (2), wherein a length of said intersection (H1) is the value of the respective periodontal bone defect and the index for the respective periodontal bone condition.

10. Method according to one or more of claims 3-8, wherein in step d) the calculation of the periodontal bone defect at the measuring position is done by calculation of a first line (A1) including the first (1) and the third landmark (3) and by calculation of a shortest distance (D1) between the second landmark (2) and said first line (A1), wherein said shortest distance (D1) is the value of the respective periodontal bone defect and the index for the respective periodontal bone condition.

11. Method according to one or more of the preceding claims, wherein in step d) the determination of the periodontal bone condition for a whole tooth (10) is done by averaging of all the values of the periodontal bone defects belonging to said tooth (10), the average of all values being the index for the respective periodontal bone condition of said tooth (10).

12. Method according to one or more of claims 1 to 10, wherein in step d) the determination of the periodontal bone condition for a whole tooth (10) is done by taking the maximum value of all the values of the periodontal bone defects belonging to said tooth (10), said maximum value being the index for the respective periodontal bone condition of said tooth (10).

13. Method according to one or more of the preceding claims, wherein in step e) the indication of the calculated periodontal bone defects and/or the indication of the calculated periodontal bone conditions, respectively, is displayed by colored sleeves around the respective teeth (10), indicating the respective position and severity of the respective periodontal bone defect.

14. Method according to one or more of the preceding claims, wherein in step e) the indication of the periodontal bone defects and/or the indication of the periodontal bone conditions, respectively, is displayed together with the respective tooth (10), wherein an area of the jawbone with the respective tooth (10) and/or a perspective of the jawbone with the respective tooth (10) and/or a magnitude of the jawbone with the respective tooth (10) are selectable.

15. Device for non-invasive semi-automatic detection and measurement of periodontal bone defects based on 3D computer tomography (CT) or digital volume tomography (DVT) scan data, said device being adapted to perform the method steps of one or more of the preceding claims.
